# EUROPEAN PATENT APPLICATION

(11) **EP 3 852 120 A1**
(43) Date of publication of application: **21.07.2021**
(21) Application number: 20152599.5
(22) Date of filing: 20.01.2020
(51) Int. Cl.: G16H 50/20, G16H 30/40, A61B 5/00

(54) **APPARATUS FOR TASK DETERMINATION DURING BRAIN ACTIVITY ANALYSIS**

(71) Applicant: Koninklijke Philips N.V., 5656 AG Eindhoven (NL)
(72) Inventor: HUIJBERS, Willem, 5656 AE Eindhoven (NL); MATTERS, Marco, 5656 AE Eindhoven (NL); LAMERICHS, Rudolf Mathias Johannes Nicolaas, 5656 AE Eindhoven (NL); VAN EE, Raymond, 5656 AE Eindhoven (NL); DENISSEN, Adrianus Johannes Maria, 5656 AE Eindhoven (NL); WESTERINK, Joanne Henriëtte Desirée Monique, 5656 AE Eindhoven (NL); LEUFKENS, Timmy Robertus Maria, 5656 AE Eindhoven (NL)
(74) Representative: Philips Intellectual Property & Standards

(57) **Abstract**

The present invention relates to an apparatus for task determination during brain activity analysis. The apparatus comprises an input unit (20), a processing unit (30), and an output unit (40). The input unit is configured to provide the processing unit with measurement data of the brain of a patient performing a task during brain activity analysis. The processing unit is configured to determine a measure of brain activity based on the measurement data of the brain. The processing unit is configured to determine:
that the patient should perform a different task to the task they are currently performing and select the different task; or
that the patient should continue performing the task that they are currently performing; or
that the patient should stop performing the task;

The determination comprises utilization of information relating to the task and the determined measure of brain activity and information relating to a plurality of reference tasks and associated plurality of reference measures of brain activity. The output unit is configured to output an indication that the patient should perform the different task, that the patient should continue performing the task, or that the patient should stop performing the task.

## Description

### FIELD OF THE INVENTION

The present invention relates to apparatus for task determination during brain activity analysis, an imaging system, and a method for task determination during brain activity analysis.

### BACKGROUND OF THE INVENTION

When brain activity is used in diagnosis or treatment navigation of various neurological and psychiatric disorders, it is not easy to select the most appropriate task paradigm (e.g. a cognitive memory task, passive rest or movie). Similar to the wide range of neuropsychological tests, a growing number of different task paradigms are employed to evoke brain activity (e.g., visual, auditory, motor, memory, language, executive, etc). The selection of the most appropriate task paradigm is thus a growing problem. Currently, a physician selects task paradigms prior to the examination and evaluates post-hoc if these were appropriate. This can result in unnecessary brain scans when multiple tasks are ordered, or additional examinations, when the data is not informative enough.

Physicians and clinical scientist have begun to request support in how better to acquire task-evoked brain imaging data, resulting in the onset of development of platforms that allow for online feature extraction on brain imaging data and research on recommender systems - see for example: McNamara Q, De La Vega A, and Yarkoni T. Developing a comprehensive framework for multimodal feature extraction. Proc ACM SIGKDD Int Conf Know1 Discov Data Min. 2017; Part F1296; 1567-1574. However, this work is in its infancy and further development and improvements are required.

### SUMMARY OF THE INVENTION

It would be advantageous to have improved means of determining tasks during brain activity analysis. The object of the present invention is solved with the subject matter of the independent claims, wherein further embodiments are incorporated in the dependent claims. It should be noted that the following described aspects and examples of the invention apply also to the apparatus for task determination during brain activity analysis, and imaging system and the method for task determination during brain activity analysis, as well as to the computer program element and a computer readable medium.

In a first aspect, there is provided an apparatus for task determination during brain activity analysis, the apparatus comprising:
- an input unit;
- a processing unit; and
- an output unit.

The input unit is configured to provide the processing unit with measurement data of the brain of a patient performing a task during brain activity analysis. The processing unit is configured to determine a measure of brain activity based on the measurement data of the brain.

The processing unit is configured to determine:
that the patient should perform a different task to the task they are currently performing and select the different task; or
that the patient should continue performing the task that they are currently performing; or
that the patient should stop performing the task;

The determination comprises utilization of information relating to the task and the determined measure of brain activity and information relating to a plurality of reference tasks and associated plurality of reference measures of brain activity. The output unit is configured to output an indication that the patient should perform the different task, that the patient should continue performing the task, or that the patient should stop performing the task.

In other words, it can be determined on the basis of the task being performed and analysis of brain measurement data such as images or imagery that the task indeed appears to be fit for purpose and that the task can be continued with further imagery being acquired in order to obtain the required degree of confidence in an understanding of the significance of the analysed brain imagery. However, it can be determined that the task being undertaken by the patient is not actually providing the desired result, and from analysis of the brain imagery a determination can be made that a different task should be made. Additionally, it can be determined that enough data have been acquired to provide the necessary result, and that the brain activity analysis can stop with the patient not having to perform the task any longer.

In this manner, an apparatus that selects tasks and adapt those tasks for diagnostic brain activity analysis is provided, such as analysis for medical diagnosis or analysis for pre-operation brain assessment, that recommends task paradigms and updates those task paradigms based on real-time analysis of brain measurement data such as imaging data. Thus, this "recommend a system" enables brain activity analysis times to be reduced, and data quality and sensitivity of brain imaging for example can be increased.

In other words, the apparatus can make a real-time analysis, i.e. decisions are made during the scanning session and that there is no post-processing or post-analysis involved in the task determination, thereby providing an improved session.

The apparatus is therefore an apparatus for task determination during acquisition of brain activity, that can acquire image data in real time for example from an MRI, EEG or other brain imaging device and determine a task for the patient to undertaker that can be for example a new cognitive, neurocognitive, neurobehavioral, psychometric, or perceptual task or be the task that they are currently undertaking. It is also to be noted that a "task" can be that the patient is asked to be in a resting state, with for examples eyes open looking at a cross on a visual display unit- VDU.

In an example, the measurement data comprise an image of the brain.

In an example, the determination that the patient should perform a different task to the task that they are currently performing and the selection of that task or that the patient should continue performing the task that they are currently performing or that the patient should stop performing the task comprises utilization of information relating to a goal of the brain activity analysis.

In an example, the goal of the brain activity analysis comprises an expected diagnosis and/or one or more brain regions to be mapped.

Thus, the "recommender system" can be constrained relative to a goal, such as make or confirm a particular diagnosis, or map brain regions prior to surgery for example. Thus, if specific brain regions required to be mapped prior to surgery, an initial task can be selected for the patient to undertake and brain imagery acquired, and that can be continued if the task is indeed targeting the correct parts of the brain, or new tasks can be recommended for the patient to undertake that would target these brain areas if the original task is not achieving the desired purpose. Additionally, medical professional may consider that a patient has a particular medical condition and a task can be provided to the patient with medical brain imagery acquired can be utilised to confirm that diagnosis when a certain degree of confidence in the result has been attained through continuation of the task. However, on the basis of the imagery being returned for the task being carried out, it can be determined that the patient could have different medical condition and therefore a different task or tasks could be provided to the patient to undertake, with the associated acquired medical brain imagery enabling different diagnosis to be made and confirmed.

In an example, the input unit is configured to provide the processing unit with new measurement data of the brain of the patient performing the different task or continuing to perform the task. The processing unit is configured to determine a measure of brain activity based on the the new measurement data of the brain. The processing unit is configured to determine:
that the patient should perform a different task to the task that they are currently performing and determine the different task; or
that the patient should continue performing the task that they are currently performing; or
that the patient should stop performing any task;

The determination comprises utilization of information relating to the task that the patient is currently performing and the determined measure of brain activity based on the new measurement data and information relating to the plurality of reference tasks and the associated plurality of reference measures of brain activity. The output unit is configured to output an indication that the patient should perform the different task to the task that they are currently performing, or that the patient should continue performing the task that they are currently performing, or that the patient should stop performing any task.

In other words, determination can be made to continue with the existing task, or change to a new task which itself may not then be appropriate and a further new task be determined to be undertaken by the patient, or determined that the brain activity analysis can stop.

In an example, the apparatus is configured to iteratively:
provide the processing unit with new measurement data of the brain of the patient;
determine a measure of brain activity based on the new measurement data; and determine that:
   the patient should perform a different task to the task that they are currently performing and determine the different task; or
   that the patient should continue performing the task that they are currently performing; and

The apparatus is configured to continue the iteration until a determination is made that the patient should stop performing any task.

In an example, the processing unit is configured to determine a medical diagnosis for the patient comprising utilization of the one or more determined measures of brain activity.

In an example, the determination of the medical diagnosis comprises utilization of the information relating to the one or more tasks performed by the patient.

In an example, the determination of the medical diagnosis comprises utilization of the plurality of reference tasks.

In an example, the determination of the medical diagnosis comprises utilization of the plurality of reference measures of brain activity.

In an example, the determination of the medical diagnosis comprises a correlation of the one or more determined measures of brain activity with one or more of the plurality of reference measures of brain activity to determine one or more reference measures of brain activity and associated information relating to the reference tasks.

In an example, the determination of the medical diagnosis comprises a selection of a reference medical diagnosis associated with at least one of the determined one or more of the plurality of reference measures of brain activity.

Thus for example, the plurality of reference tasks and associated plurality of reference measures of brain activity can be a library or ontology of task evoked brain activity patterns associated with diagnosis and treatment information. Thus, the acquired and analysed brain imagery can be correlated with analysed brain imagery in the library, and from the associated diagnosis of the best match it can be determined what the probable diagnosis for the patient is. This diagnosis can be augmented through information relating to the actual task or tasks that the patient has been undertaking, for example brain activity pattern resulting from analysis of brain imagery may match with two brain activity patterns in the library, where one of those activity patterns stored related to the same task that the patient was undertaking with the different one relating to a different task. Therefore, it is unable to be determined that the matching pattern with matching task, is the better match, enabling the associated medical diagnosis with the reference data to be used in providing a diagnosis the patient now undertaking the test.

In an example, the determination that the patient should perform a different task to the task they are currently performing and the determination of the different task or that the patient should continue performing the task that they are currently performing or that the patient should stop performing the task comprises implementation of a pattern matching algorithm to determine correlations between the determined measure of brain activity and the plurality of reference measures of brain activity.

In an example, the pattern matching algorithm comprises a nearest neighbor classifier.

In a second aspect, there is provided an imaging system, comprising:
- a brain imaging device (110); and
- an apparatus (10) according to any of claims 1-14;

The brain imaging device is configured to acquire at least one image of the brain of a patient performing a task during brain activity analysis and provide the at least one image to the apparatus. The apparatus is configured to output an indication that the patient should perform a selected different task to the task that they are currently performing, or that the patient should continue performing the task that they are currently performing, or that the patient should stop performing the task that they are currently performing or stop performing any task on the basis of one or more images of the at least one image of the brain of the patient.

In a third aspect, there is provided a method for task determination during brain activity analysis, the method comprising:
a) providing a processing unit with measurement data of the brain of a patient performing a task during brain activity analysis,
b) determining by the processing unit a measure of brain activity based on the measurement data of the brain;
c) determining by the processing unit:
   that the patient should perform a different task to the task they are currently performing and determine the different task; or
   that the patient should continue performing the task that they are currently performing; or
   that the patient should stop performing the task;
   wherein, the determining comprises utilizing information relating to the task and the determined measure of brain activity and information relating to a plurality of reference tasks and associated plurality of reference measures of brain activity; and
d) outputting by an output unit an indication that the patient should perform the different task, that the patient should continue performing the task, or that the patient should stop performing the task.

According to another aspect, there is provided a computer program element controlling one or more of the apparatuses or systems as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for task determination during brain activity analysis;
Fig. 2 shows a schematic set up of an example of an imaging system;
Fig. 3 shows a method apparatus for task determination during brain activity analysis; and
Fig. 4 shows a representation of a workflow of task determination during brain activity analysis.

### DETAILED DESCRIPTION OF EMBODIMENTS

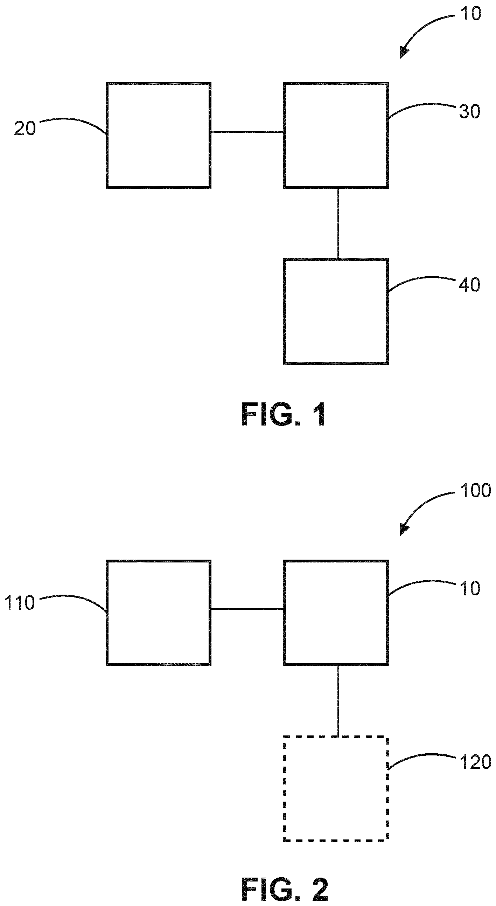

Fig. 1 shows an example of an apparatus for task determination during brain activity analysis. The apparatus comprises an input unit 20, a processing unit 30, and an output unit 40. The input unit is configured to provide the processing unit with measurement data of the brain of a patient performing a task during brain activity analysis. The processing unit is configured to determine a measure of brain activity based on the measurement data of the brain. The processing unit is configured to determine: that the patient should perform a different task to the task that is currently being performed and select the different task; or that the patient should continue performing the task that is currently being performed; or that the patient should stop performing the task; the determination comprises utilization of information relating to the task and the determined measure of brain activity and information relating to a plurality of reference tasks and associated plurality of reference measures of brain activity. The output unit is configured to output an indication that the patient should perform the different task, that the patient should continue performing the task, or that the patient should stop performing the task.

In an example, the plurality of reference tasks and associated plurality of reference measures of brain activity can be a library or ontology of task evoked brain activity patterns associated with diagnosis and treatment information. Thus, the patient undertakes a task and at the same time brain images are acquired and analysed. Then, the task and associated analysis can be used to interrogate this library to determine if task being undertaken is fit for purpose, or if different task may be better suited for the purposes of the brain activity acquisition and analysis. Then, the determination can be made to continue the existing task or change to a new task or finalize the acquisition and analysis session. Thus, for example a measured brain activity pattern resulting from brain imagery and the associated task can be used to interrogate a library of task information with associated brain activity patterns, from which the new task can be determined or a determination made that the existing task is fit for purpose. You can also be determined that enough information has been acquired to stop the analysis.

In an example, the image of the brain is acquired by an MRI, EEG, ECOG, MEG, OPM, PET, fNIRS, fUS brain imaging device.

The determination that the patient should stop performing the task means that the patient should stop performing any task, and as such is different to the determination that the patient should perform a selected different task. Thus, the determination that the patient should stop performing the task means that the brain activity acquisition and analysis is complete.

According to an example, the measurement data comprises an image of the brain.

According to an example, the determination that the patient should perform a different task to the task that they are currently performing and the selection of that task or that the patient should continue performing the task that they are currently performing or that the patient should stop performing the task comprises utilization of information relating to a goal of the brain activity analysis.

According to an example, the goal of the brain activity analysis comprises an expected diagnosis and/or one or more brain regions to be mapped.

According to an example, the input unit is configured to provide the processing unit with new measurement data of the brain of the patient performing the different task or continuing to perform the task. The processing unit is configured to determine a measure of brain activity based on the the new measurement data of the brain. The processing unit is configured to determine: that the patient should perform a different task to the task that they are currently performing and determine the different task; or that the patient should continue performing the task that they are currently performing; or that the patient should stop performing any task; the determination comprises utilization of information relating to the task that the patient is currently performing and the determined measure of brain activity based on the new measurement data and information relating to the plurality of reference tasks and the associated plurality of reference measures of brain activity. The output unit is configured to output an indication that the patient should perform the different task to the task that they are currently performing, or that the patient should continue performing the task that they are currently performing, or that the patient should stop performing any task.

In an example, the new measurement data comprises a new image of the brain.

According to an example, the apparatus is configured to iteratively:
provide the processing unit with new measurement data of the brain of the patient;
determine a measure of brain activity based on the new measurement data; and
   determine that:
   the patient should perform a different task to the task that they are currently performing and determine the different task; or
   that the patient should continue performing the task that they are currently performing; and

The apparatus is configured to continue the iteration until a determination is made that the patient should stop performing any task.

In an example, the new measurement data comprises a new image of the brain.

According to an example, the processing unit is configured to determine a medical diagnosis for the patient comprising utilization of the one or more determined measures of brain activity.

According to an example, the determination of the medical diagnosis comprises utilization of the information relating to the one or more tasks performed by the patient.

According to an example, the determination of the medical diagnosis comprises utilization of the plurality of reference tasks.

According to an example, the determination of the medical diagnosis comprises utilization of the plurality of reference measures of brain activity.

According to an example, the determination of the medical diagnosis comprises a correlation of the one or more determined measures of brain activity with one or more of the plurality of reference measures of brain activity to determine one or more reference measures of brain activity and associated information relating to the reference tasks.

According to an example, the determination of the medical diagnosis comprises a selection of a reference medical diagnosis associated with at least one of the determined one or more of the plurality of reference measures of brain activity.

According to an example, the determination that the patient should perform a different task to the task they are currently performing and the determination of the different task or that the patient should continue performing the task that they are currently performing or that the patient should stop performing the task comprises implementation of a pattern matching algorithm to determine correlations between the determined measure of brain activity and the plurality of reference measures of brain activity.

According to an example, the pattern matching algorithm comprises a nearest neighbor classifier.

Fig. 2 shows an example of an imaging system 100, where optional features are shown as a dashed box. The imaging system comprises a brain imaging device 110, and an apparatus 10 as described with respect to Fig. 1. The brain imaging device is configured to acquire at least one image of the brain of a patient performing a task during brain activity analysis and provide the at least one image to the apparatus. The apparatus is configured to output an indication that the patient should perform a selected different task to the task that they are currently performing, or that the patient should continue performing the task that they are currently performing, or that the patient should stop performing the task that they are currently performing or stop performing any task on the basis of one or more images of the at least one image of the brain of the patient.

In an example, the imaging system comprises a library of information relating to a plurality of reference tasks and associated plurality of reference measures of brain activity, and wherein the processing unit of the apparatus is configured to interrogate library.

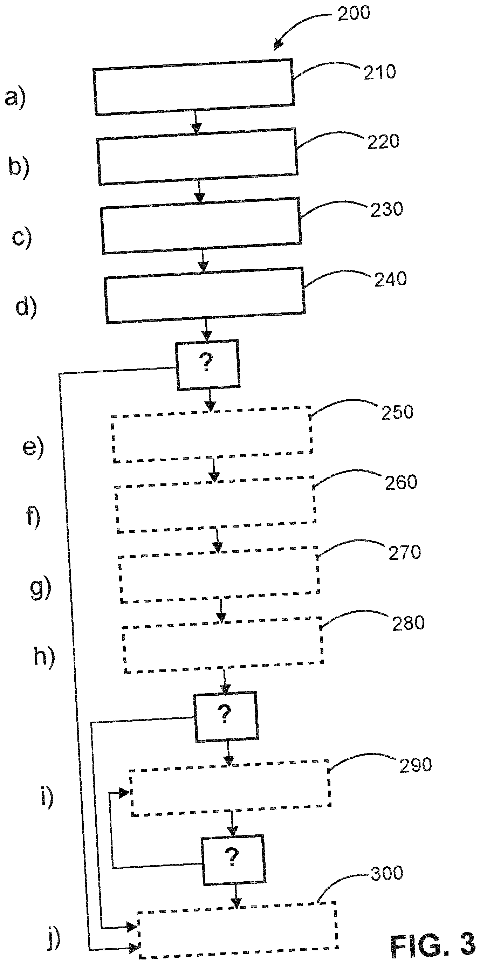

Fig. 3 shows a method 200 for task determination during brain activity analysis in its basic steps, where optional steps are shown with dashed boxes. The method comprises:
in a providing step 210, also referred to as step a), providing a processing unit with measurement data of the brain of a patient performing a task during brain activity analysis,
in a determining step 220, also referred to as step b), determining by the processing unit a measure of brain activity based on the measurement data of the brain;
in a determining step 230, also referred to as step c), determining by the processing unit:
   that the patient should perform a different task to the task they are currently performing and determine the different task; or
   that the patient should continue performing the task that they are currently performing; or
   that the patient should stop performing the task;
   in step c) the determining comprises utilizing information relating to the task and the determined measure of brain activity and information relating to a plurality of reference tasks and associated plurality of reference measures of brain activity; and
   in an outputting step 240, also referred to as step d), outputting by an output unit an indication that the patient should perform the different task, that the patient should continue performing the task, or that the patient should stop performing the task.

In an example, the measurement data comprises an image of the brain.

In an example, the measurement data of the brain is acquired by an MRI, EEG, ECOG, MEG, OPM, PET, fNIRS, or fUS brain imaging device.

In an example, step c) comprises utilizing information relating to a goal of the brain activity analysis.

In an example, the goal of the brain activity analysis comprises an expected diagnosis and/or one or more brain regions to be mapped.

In an example, the method comprises:
in a providing step 250, also referred to as step e), providing the processing unit with new measurement data of the brain of the patient performing the different task or continuing to perform the task;
in a determining step 260, also referred to as step f), determining by the processing unit a measure of brain activity based on the new measurement data of the brain;
in a determining step 270, also referred to as step g), determining by the processing unit:
   that the patient should perform a different task to the task that they are currently performing and determine the different task; or
   that the patient should continue performing the task that they are currently performing; or
   that the patient should stop performing any task;
   in step g) the determining comprises utilizing information relating to the task that the patient is currently performing and the determined measure of brain activity based on the new measurement data and information relating to the plurality of reference tasks and the associated plurality of reference measures of brain activity; and
   in an outputting step 280, also referred to as step h), outputting by the output unit an indication that the patient should perform the different task to the task that they are currently performing, or that the patient should continue performing the task that they are currently performing, or that the patient should stop performing any task.

In an example, the new measurement data comprises a new image of the brain.

In an example, the method comprises:
in an iterator step 290, also referred to as step i), iteratively providing the processing unit with new measurement data of the brain of the patient, and determining a measure of brain activity, and determining that the patient should perform a different task to the task that they are currently performing and determine the different task or that the patient should continue performing the task that they are currently performing, and wherein continue the iteration until determining that the patient should stop performing any task.

In an example, the new measurement data comprises a new image of the brain.

In an example, the method comprises:
in a determining step 300, also referred to as step j), determining by the processing unit configured a medical diagnosis for the patient comprising utilizing the one or more determined measures of brain activity.

In an example, step j) comprises utilizing the information relating to the one or more tasks performed by the patient.

In an example, step j) comprises utilizing the plurality of reference tasks.

In an example, step j) comprises utilizing the plurality of reference measures of brain activity.

In an example, step j) comprises correlating the one or more determined measures of brain activity with one or more of the plurality of reference measures of brain activity to determine one or more reference measures of brain activity and associated information relating to the reference tasks.

In an example, step j) comprises selecting a reference medical diagnosis associated with at least one of the determined one or more of the plurality of reference measures of brain activity.

In an example, step c) comprises implementing a pattern matching algorithm to determine correlations between the determined measure of brain activity and the plurality of reference measures of brain activity.

In an example, step g) comprises implementing the pattern matching algorithm to determine correlations between the determined measure of brain activity and the plurality of reference measures of brain activity.

In an example, the pattern matching algorithm comprises a nearest neighbor classifier.

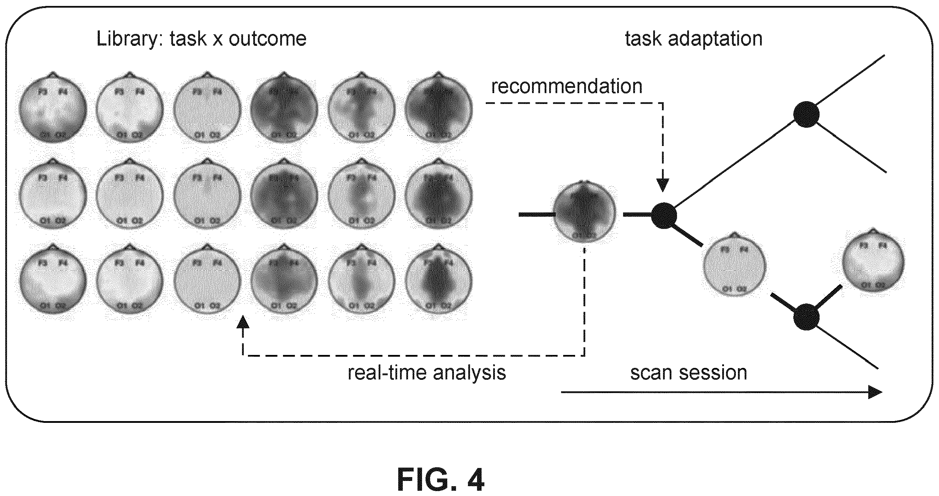

The apparatus for task determination during brain activity analysis, imaging system and method for task determination during brain activity analysis are now described in specific further detail, where reference is made to Fig. 4.

Fig. 4 shows a representation of a workflow of task determination during brain activity analysis. The workflow applies to an apparatus that receives data from a brain-imaging device (e.g. MRI, EEG, ECOG, MEG, OPM, PET, fNIRS, fUS) with local computational resources or connectivity for real-time analysis of (functional) imaging data. A library/ontology of task-evoked brain activity patterns by outcome (diagnosis / treatment) is also utilized or interrogated, and the apparatus in effect operates as a real-time recommender system that based on the measured brain activity patterns recommends task adaptations, switching or stopping data acquisition.

Continuing with Fig. 4 this illustrates task adaptation/selection based on real-time analysis of electroencephalography (EEG) data. On the left a library with images of task x outcome is shown. On the right, an adaptive path found during image acquisition based on two "recommendations" to adapt (or switch) the task is shown. Similar to task adaptation, the system recommends stopping acquisition when a pattern of activity has been evoked with enough confidence (or a desired reliability). For example, an evoked potential on O1 and O4 using EEG with a desired confidence or reliability. Another example, could be based on functional MRI, using %-signal change in brain regions of interest (fusiform face area, etc) or connectivity strength of cortical network. In that case, the EEG images above would be replaced by brain activity/connectivity maps estimated using task, movie-based or resting-state fMRI.

Continuing with Fig. 4, three different task scans are made. The system then made two recommendations to switch/change scans based on the real-time analysis. The left part of the figure shows the "path" recommended by the system (= equal to path by operator). These recommendations can be constrained relative to an outcome (or goal) that has to be determined. For example, does patient Y have a mental disorder Q, for example schizophrenia? After scan 1, when enough data is gathered, the system recommends task-2 (or scan 2) and similarly task-3 (or scan 3). The result of these different scans might all convergence on the same diagnosis, and immediately aid in determination a specific sub-type or treatment option. Alternatively, they might help gather evidence for an alternative diagnosis (bipolar, depression etc). In each step/recommendation, the scans are matched to a library of reference scans and associated data such as a library/ontology of task-evoked brain activity patterns by outcome (diagnosis / treatment). Depending on the scanning methodology used (MRI, EEG, etc) and exact parameters of the scans, this can involve different algorithms and measurements. A relatively straightforward implementation is to use a nearest neighbor classifier to match the pattern to previously acquired scans in the library. With respect to recommending that acquisition is stopped, the following provides more specific detail, where EEG is provided as an example. For example, in EEG, the same condition is acquired multiple times (i.e. data is acquired for the patient performing the same task multiple times). After the scan, a statistical brain map is made. Thus, task-data is gathered until activity in a predefined region (= an electrode 01) reaches a statistically significant threshold, which can be considered to be when a certain degree of confidence, reliability in the data has been reached, where this can be considered to be equivalent to a "signal to noise ratio".

As detailed with respect to Fig. 4, EEG or fMRI are two possible imaging modalities. The apparatus or "recommender-system" can therefore also be implemented on a magnetic resonance imaging (MRI) scanner. The apparatus/system can be built using existing task paradigms and conventional imaging sequences, for example focusing on the tasks paradigms used in the human connectome project (HCP). The real-time analysis of brain imaging data utilizes the following components:
A brain-imaging device to acquire new scans (e.g. MRI, EEG, etc.), but described here with respect to MRI.

A library to match the new scans to previously acquired data

A computational core to reconstruct, analyze and match scans in real-time.

A recommender system to evaluate new scans and, library scans

First, the brain-imaging device, for example an MRI scanner that enables acquisition of functional MRI consistent with the HCP protocol, can utilize echo-planar imaging at 3 Tesla. In addition, the patients should be able to perform the HCP tasks while inside the MRI scanner. Therefore, they should be able to view a computer screen, hear sounds via a headphone and press buttons on an MRI-compatible keypad.

Secondly, a library populated with reference scans, using similar functional MRI data, collected in the various studies of HCP studies is available. These scans are available from public data repositories and contain data from healthy adults and patients with different psychiatric and neurological brain disorders.

Third, once MRI data collection starts, the imaging data are processed by the computation core in real-time. This includes reconstruction, pre-processing and task-paradigm dependent analyses. The reconstruction resolves the transformation of MRI data from the Fourier to the spatial domain. The preprocessing includes slice-time correction, realignment and normalization to the atlas space. Several, quality control steps can be deployed in reconstruction and preprocessing. For instance, if the realignment requires too much correction, individual volumes can be disregarded. Optionally, the recommender system can use the quality control to recommend additional fMRI acquisition with the current task. After preprocessing, the fMRI data is analyzed in relation to various the task-paradigms. Using the Hariri-task as an example, the computational core calculates the signal difference between task condition 1 (emotional faces) and task condition 2 (abstract shape). This signal difference can be calculated for the entire brain or only pre-determined regions of interest. Instead of a signal difference, task-paradigm dependent analyses can also use methods that examine coherence between regions of interest. The task-paradigm dependent analyses result in image-derived metrics. These metrics are subsequently used to compare the new data to previously acquired data in the library.

Fourth, the recommender system makes a comparison between the new data and scans in the library. When the goal is classification (or diagnosis), the recommender identifies the most similar patient(s) in the library based on based on image-derived metric. Subsequently, it would recommend acquisition of those tasks (or sequences) that have been acquired in the most similar patient(s).

Therefore, in summary the apparatus/system analyses the functional MRI signal in a predefined brain region during specific task conditions or passive-rest. Using a passive-rest condition, connectivity analyses are used to estimate coherence between pre-defined brain regions. Based on these connectivity estimates the apparatus can recommend specific follow-up scans. The resting-state fMRI scan can for example be followed by a specific task paradigm requiring the patient to view and respond to images with emotional faces. In this case, the real-time analysis can extract %-signal from pre-defined brain regions in the visual cortex, fusiform face area and amygdala. Based on the fMRI signal, the system can find evidence for abnormal activity within these pre-defined brain regions, or again using connectivity analyses, quantify coherence between pre-defined brain regions. This can result in enough evidence in a recommend to halt fMRI acquisition, or recommend that a third scan be taken or that a different task be performed and fMRI date be acquired.

The apparatus, or if it includes the image acquisition device the system, can be built using existing task paradigms and conventional imaging sequences, for example focusing on the tasks paradigms used in the human connectome project. Therefore, the apparatus/system can be built using the various Human Connectome Project (HCP) projects on brain diseases including, mental illness, anxiety and depression, treatment of resistant depression, early psychosis, psychotic psychopathology, epilepsy, frontal temporal dementia and Alzheimer's Disease subtypes - see Barch DM, Burgess GC, Harms MP, Petersen SE, Schlaggar BL, Corbetta M, et al. Function in the human connectome: Task-fMRI and individual differences in behavior. Neuroimage. 2013; 80: 169-189. The Human Connectome Project (HCP) has selected and developed task paradigms to assess as many different neural systems as it is feasible within the time that the project had available to scan each participant. These "functional localizer" tasks include measures of primary sensory processes (e.g., vision, motor function) and as many different cognitive and affective processes as possible, including stimulus category representations, working memory, episodic memory, language processing, emotion processing, and decision-making. The HCP tasks also include a "Hariri" task on emotional processing. See: https://www.humanconnectome.org/study/hcp-young-adult/document/q1-data-release/task-fMRI. The library referred to above, is then populated with reference scans from the HCP studies (these are available from public data repositories), where the reference scans are associated information can be patterns of activity in predefined regions of the brain for certain tasks. Once data collection starts, the online-acquired imaging data is transformed to the atlas-space and matched to templates in the library. Once a desired signal-to-noise ratio is achieved, the system can recommend to stop acquisition. An apparatus or system to optimally select different task-paradigms, can be built using task-paradigms used in the various HCP projects on brain diseases including, mental illness, anxiety and depression, treatment of resistant depression, early psychosis, psychotic psychopathology, epilepsy, frontal temporal dementia and Alzheimer's Disease subtypes. See for example: Gordon EM, Laumann TO, Gilmore AW, Newbold DJ, Greene DJ, Berg JJ, et al. Precision Functional Mapping of Individual Human Brains. Neuron. 2017; 95: 791-807, Glasser MF, Smith SM, Marcus DS, Andersson J, Auerbach EJ, Behrens TEJ, et al. The Human Connectome Project's Neuroimaging Approach. Nat Neurosci. 2016; 19: 1175-1187, and Harms MP, Somerville LH, Ances BM, Andersson J, Barch DM, Bastiani M, et al. Extending the Human Connectome Project across ages: Imaging protocols for the Lifespan Development and Aging projects. Neuroimage. 2018; 183: 972-984.

The apparatus and system can be built that continuously keeps learning using for example an online secure platform that combines or integrates data from multiple sites in a safe, private and compliant manner. The recommender can be on a brain imaging acquisition system. Once in place, the recommender system initially can only suggest scans and task paradigms it knows (from for example the HCP). Yet, when a user acquires with an unknown scan following a known (HCP) scan, the recommender system can learn new pairs. Subsequently, the recommender can suggest this unknown scan as the preferred follow-up. As these news scans become part of the recommender, the frequency of the recommendation provides indication to the system-developer that real-time analysis of these new scans is preferred. After these news scans are part of the real-time analysis pipeline, the recommenders could suggest these new scans after as an initial scan or make learn suggestions after the new scan.

It is to be noted that rather than transforming image data to the atlas-space and match to templates in the library, alternatively the library, or subset of template patterns of brain activity used from the library, can be transformed to native space using anatomical reference scans. This would require an anatomical brain scan and more computation prior to the functional brain imaging, but reduce the real-time computation requirements.

Therefore, for example when a new scan is acquired for a new patient, a similarity/distance measure is calculated between the new patient and data in library with reference scans (or a subset of this library). If schizophrenia is suspected, the problem can be constrained by calculating the similarity between the new brain versus reference scans from a set of schizophrenia patients and healthy adults. It is then determined if the pattern is more similar to healthy adults or patients with schizophrenia and based on the similarity, the system makes a recommendation. There are many different algorithms to calculate similarity (for example the absolute difference or a pearson correlation).

A "normalization" process can be utilized, because each person has a uniquely shaped brain, regardless of the differences in activity. Therefore, each brain scan is transformed into a shared "atlas" space (this can be considered to be a sort of spatial normalization). The atlas-space also has a coordinate system. In the system, it is known for example that coordinate x,y,z is brain regions Y, and the HCP task maps are also transformed to this space. In atlas-space, it is then simply to compare the activity in region Y between two people. Two methods can be used to provide this spatial normalization:
Method 1 (native2atlas): the apparatus/system analyses the scans as data comes in and transforms the new data to the atlas space. Thus, the apparatus/system does the transformation from native space (patients) to the template space on the fly and matches the new data in atlas space.
Method 2 (altas2native): The apparatus/system brings a set of scans from the library to the native space of patients. This inverse transform can yield better results (less loss of data). However, more computationally is required, because the entire library cannot be transformed. Thus, this would require prior knowledge on which subset templates in the library are important. Yet, if an anatomical scan is available or made prior to the task fMRI scans, these inverse atlas2native transforms can be done before. In that case, it would actually reduce the real-time computational burden, as it is no longer needed to perform real-time spatial normalization, and the apparatus/system can immediately calculate similarity.

The following are features of the recommender system:
In a simple straightforward case, the recommender system can suggest to increase the acquisition time to identify signal a specific electrode, in specific brain region or cortical network with a desired signal strength.

When a desired signal from an electrode, brain area or network, shows too much noise, the recommender can identify the sources of noise (e.g. motion, breathing, operator error, system instability) and suggest improvements for further acquisition.

In the setting of naturalistic paradigms, including movies, the recommender system can be used to recommend movies with specific content in order to identify network of dissociate subtypes of patients.

Mapping of task-evoked activity near a brain tumour. When functional magnetic resonance imaging (fMRI) is used for pre-surgical planning in tumour patients, typically a language-task is used, regardless of exact location of the brain tumour.

In developmental disorders, different types of language tasks can be efficiently implemented with EEG for diagnosis.

In another exemplary embodiment, a computer program or computer program element is provided that is characterized by being configured to execute the method steps of the method according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus for task determination during brain activity analysis, the apparatus comprising:
- an input unit (20);
- a processing unit (30); and
- an output unit (40);
wherein, the input unit is configured to provide the processing unit with measurement data of the brain of a patient performing a task during brain activity analysis;
wherein, the processing unit is configured to determine a measure of brain activity based on the measurement data of the brain;
wherein, the processing unit is configured to determine:
that the patient should perform a different task to the task they are currently performing and select the different task; or
that the patient should continue performing the task that they are currently performing; or
that the patient should stop performing the task;
wherein the determination comprises utilization of information relating to the task and the determined measure of brain activity and information relating to a plurality of reference tasks and associated plurality of reference measures of brain activity; and
wherein the output unit is configured to output an indication that the patient should perform the different task, that the patient should continue performing the task, or that the patient should stop performing the task.

2. Apparatus according to claim 1, wherein the measurement data comprises an image of the brain.

3. Apparatus according to any of claims 1-2, wherein the determination that the patient should perform a different task to the task that they are currently performing and the selection of that task or that the patient should continue performing the task that they are currently performing or that the patient should stop performing the task comprises utilization of information relating to a goal of the brain activity analysis.

4. Apparatus according to claim 3, wherein the goal of the brain activity analysis comprises an expected diagnosis and/or one or more brain regions to be mapped.

5. Apparatus according to any of claims 1-4, wherein the input unit is configured to provide the processing unit with new measurement data of the brain of the patient performing the different task or continuing to perform the task, wherein the processing unit is configured to determine a measure of brain activity based on the new measurement data of the brain, wherein the processing unit is configured to determine:
that the patient should perform a different task to the task that they are currently performing and determine the different task; or
that the patient should continue performing the task that they are currently performing; or
that the patient should stop performing any task;
wherein the determination comprises utilization of information relating to the task that the patient is currently performing and the determined measure of brain activity based on the new measurement data and information relating to the plurality of reference tasks and the associated plurality of reference measures of brain activity; and
wherein the output unit is configured to output an indication that the patient should perform the different task to the task that they are currently performing, or that the patient should continue performing the task that they are currently performing, or that the patient should stop performing any task.

6. Apparatus according to any of claims 1-5, wherein the apparatus is configured to iteratively:
provide the processing unit with new measurement data of the brain of the patient;
determine a measure of brain activity based on the new measurement data; and
determine that:
the patient should perform a different task to the task that they are currently performing and determine the different task; or
that the patient should continue performing the task that they are currently performing; and
wherein the apparatus is configured to continue the iteration until a determination is made that the patient should stop performing any task.

7. Apparatus according to any of claims 1-6, wherein the processing unit is configured to determine a medical diagnosis for the patient comprising utilization of the one or more determined measures of brain activity.

8. Apparatus according to claim 7, wherein the determination of the medical diagnosis comprises utilization of the information relating to the one or more tasks performed by the patient.

9. Apparatus according to any of claims 7-8, wherein the determination of the medical diagnosis comprises utilization of the plurality of reference tasks.

10. Apparatus according to any of claims 7-9, wherein the determination of the medical diagnosis comprises utilization of the plurality of reference measures of brain activity.

11. Apparatus according to claim 10, wherein the determination of the medical diagnosis comprises a correlation of the one or more determined measures of brain activity with one or more of the plurality of reference measures of brain activity to determine one or more reference measures of brain activity and associated information relating to the reference tasks.

12. Apparatus according to claim 11, wherein the determination of the medical diagnosis comprises a selection of a reference medical diagnosis associated with at least one of the determined one or more of the plurality of reference measures of brain activity.

13. Apparatus according to any of claims 1-12, wherein the determination that the patient should perform a different task to the task they are currently performing and the determination of the different task or that the patient should continue performing the task that they are currently performing or that the patient should stop performing the task comprises implementation of a pattern matching algorithm to determine correlations between the determined measure of brain activity and the plurality of reference measures of brain activity.

14. Apparatus according to claim 13, wherein the pattern matching algorithm comprises a nearest neighbor classifier.

15. An imaging system (100), comprising:
- a brain imaging device (110); and
- an apparatus (10) according to any of claims 1-14;
wherein, the brain imaging device is configured to acquire at least one image of the brain of a patient performing a task during brain activity analysis and provide the at least one image to the apparatus, and
wherein, the apparatus is configured to output an indication that the patient should perform a selected different task to the task that they are currently performing, or that the patient should continue performing the task that they are currently performing, or that the patient should stop performing the task that they are currently performing or stop performing any task on the basis of one or more images of the at least one image of the brain of the patient.

16. A method (200) for task determination during brain activity analysis, the method comprising:
a) providing (210) a processing unit with measurement data of the brain of a patient performing a task during brain activity analysis,
b) determining (220) by the processing unit a measure of brain activity based on the measurement data of the brain;
c) determining (230) by the processing unit:
that the patient should perform a different task to the task they are currently performing and determine the different task; or
that the patient should continue performing the task that they are currently performing; or
that the patient should stop performing the task;
wherein, the determining comprises utilizing information relating to the task and the determined measure of brain activity and information relating to a plurality of reference tasks and associated plurality of reference measures of brain activity; and
d) outputting (240) by an output unit an indication that the patient should perform the different task, that the patient should continue performing the task, or that the patient should stop performing the task.

17. A computer program element for controlling an apparatus according to any of claims 1-14 or a system according to claim 15, which when executed by a processor is configured to carry out the method of claim 16.
